(19) Europäisches Patentamt
European Patent Office
Office européen des brevets

(11) Publication number: 0 436 481 A1

(12) EUROPEAN PATENT APPLICATION

(21) Application number: 90870004.0

(22) Date of filing: 04.01.90

(51) Int. Cl.[5]: C07D 207/20, C07D 211/70, //A23L1/226

(43) Date of publication of application: 10.07.91 Bulletin 91/28

(84) Designated Contracting States: BE CH DE FR LI NL

(71) Applicant: RIJKSUNIVERSITEIT GENT Faculteit van de Landbouwwetenschappen
Laboratorium voor Organische Scheikunde
Coupure Links, 653
B-9000 Gent(BE)

(72) Inventor: De Kimpe, Norbert, Dr. ir.
Lodries 7
B-9210 Heusden(BE)
Inventor: Stevens, Chris
Molenstraat 96
B-9241 Schelderode(BE)
Inventor: Schamp, Niceas, Prof. Dr.
Mieregoedstraat 25
B-9720 De Pinte(BE)

(74) Representative: Vanderperre, Robert et al
Bureau Vander Haeghen S.A. Rue Colonel Bourg 108 A
B-1040 Bruxelles(BE)

# (54) Process for preparing 2-acetyl-1-aza-1-cycloalkenes useful as flavor components for bread and rice.

(57) Straightforvard and inexpensive syntheses of 2-acetyl-1-aza-1-cycloalkenes usefull as bread and rice flavor components, i.e. 2-acetyl-1,4,5,6-tetrahydropyridine and 2-acetyl-1-pyrroline, respectively, comprise the following steps :

- oxidation of cyclic amines to cyclic imines;
- subsequent cyanation of cyclic amines to 2-cyano-1-azacycloalkanes ;
- oxidation of 2-cyano-1-azacycloalkanes to 2-cyano-1-aza-1-cycloalkenes;
- synthesis of 2-acetyl-1-aza-1-cycloalkenes by a Grignard reaction.

These syntheses give rise to the title compounds, free of side products, in a way easily amenable to industrial production.

EP 0 436 481 A1

# PROCESS FOR PREPARING 2-ACETYL-1-AZA-1-CYCLOALKENES USEFULL AS FLAVOR COMPONENTS FOR BREAD AND RICE.

The present invention relates to a process for preparing 2-acetyl-1-aza-1-cycloalknes usefull as flavor components for bread and rice.

Flavor and aroma play a pronounced role in the food industry. Consumer demand has led the flavor manufacturers to create new and improved flavors. Numerous food products are being upgraded to "value-added" products by the incorporation of flavorings with appropriate functional ingredients. In several food products, the natural inherent flavors of the food ingredients are not strong enough to deliver a good final product. This fact creates the need for an enhancer in order to boost the flavor or the foodstuff.

A variety of heterocyclic compounds are distributed in food flavors. These compounds originate from enzymatic reactions of several substrates ( e.g. polyphenols, lipids, and proteins ) and non-enzymatic browning reactions between α-amino acids and reducing sugars. The latter process is referred to as the Maillard reaction, which gaines in importance with increasing temperatures.

During the Maillard reaction of L-proline and reducing sugars, more than one hundred proline-specific compounds are formed, depending on the reaction conditions and the sugars used [B. Helak, E. Kersten, K. Spengler, R. Tressl, D. Rewicki, J. Agric. Food Chem., vol.37, 405 (1989); B. Helak, K. Spengler, R. Tressl, D. Rewicki, J. Agric. Food Chem., 37, 400 (1989)]. Many of these compounds, such as 2-acetyl-1,4,5,6-tetrahydropyridine 3, display cracker-like odor characteristics. This compound has been identified in freshly baked bread [I.R. Hunter, M.K. Walden, J.R. Scherer, R.E. Lundin, Cereal Chem., vol 46, 189 (1969)] and in the crust of freshly baked rye bread [P. Schieberle, W. Grosch, Z. Lebensm. Unters. Forsch., vol 178, 479 (1984); P. Schieberle, W. Grosch, Z. Lebensm. Unters. Forsch., vol 177, 173 (1983)].

Although 2-acetyl-1,4,5,6-tetrahydropyridine 3 is a rather labile compound, it is currently considered as the most significant bread flavor component. US-A-3620771 and US-A-3725425 disclose that 2-acetyl-1,4,5,6-tetrahydropyridine 3 is available by synthesis in an unspecified yield via thermal condensation of proline with 1,3-dihydroxy-2-propanone in the presence of sodium bisulfite. An improved synthesis was published later, utilizing an expensive rhodium catalyst and a silver reagent [G. Büchi, H. Wüest, J. Org. Chem., vol 36, 609 (1971)].

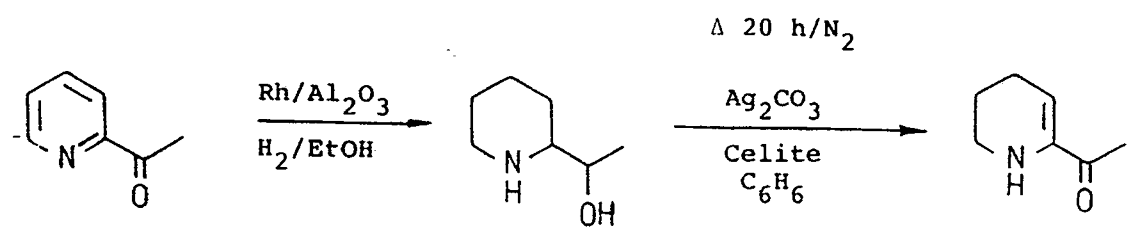


1 | 2 (78%) | 3 (57%)

2-Acetyl-1,4,5,6-tetrahydropyridine 3 has been stabilized as the bisulfite adduct and as the hydrochloride. Both derivatives were used as flavoring agents for bread and bakery products. It displays a characteristic and extremely strong cracker-like flavor. Only two syntheses of 2-acetyl-1,4,5,6-tetrahydropyridine have been disclosed in the literature (vide supra). The second synthesis of 2-acetyl-1,4,5,6-tetrahydropyridine 3 entails a well-established procedure (overall yield of 44% starting from the relatively expensive 2-acetylpyridine 1), but this synthesis is less attractive for industrial purposes because of the use of the extremely expensive rhodium catalyst and the use of an excess of very expensive silver salts [G. Büchi, H. Wüest, J. Org. Chem., vol.36, 609 (1971)].

The analogous 2-acetyl-1-pyrroline 6 has a similar potent cracker-like flavor and is considered as the most important flavor component of cooked rice. It has been identified and isolated from different varieties of cooked rice [R.G. Buttery, L.C. Ling, B.O. Juliano, Chem. Ind., 958 (1982); R.G. Buttery, L.C. Ling, B.O. Juliano, J.G. Turnbaugh, J. Agric. Food Chem., vol.31, 823 (1983); R.G. Buttery, L.C. Ling, T.R. Mon, J. Agric. Food Chem., vol.34, 112 (1986)] and the crust of wheat and rye bread [P. Schieberle, W. Grosch, J. Agric. Food Chem., vol.35, 252 (1987); P. Schieberle, w. Grosch, Z. Lebensm. Unters Forsch., vol. 180, 474 (1985)]. It is remarkable that 2-acetyl-1-pyrroline 6 has been found in pandam leaves (Pandanus amaryllifolius Roxb.)[R.G. Buttery, B.O. Juliano, L.C. Ling, Chem. Ind., 478 (1983)]. This fact explains that it has long been the practice in India and other parts of Asia to use leaves of Pandanus species in the cooking of

common rices to impart a resemblance of the aroma of the more costly scented rice.

U.S. Pat. 4,522,838 discloses the sole known synthetic route to 2-acetyl-1-pyrroline.

The synthesis entails hydrogenation of 2-acetylpyrrole 4 with rhodium on alumina, followed by oxidation of the resulting aminoalcohol 5 by means of an excess of silver carbonate (absorbed on celite) in benzene.

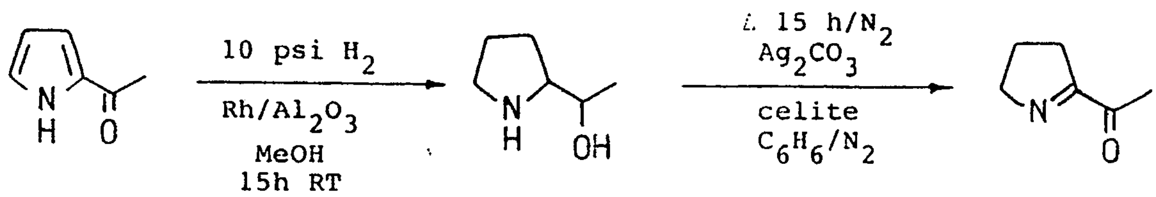


4 5 6 (10% overall)

2-Acetyl-1-pyrroline 6 has been used in flavoring foods, particularly in imparting a scented rice flavor to foods. The drawback of this synthesis of the rice flavor component 6 is the use of the very expensive reagents, the low overall yield of 10%, the use of toxic chemicals (e.g. benzene) and the virtually inaccessibility of the compound on a larger scale. Indeed, according to the patented procedure mentioned above, 2-acetyl-1-pyrroline 6 was isolated and purified by preparative gas chromatography, which entails at best subgram quantities.

It is clear from the above mentioned results that 2-acetyl-,4,5,6-tetrahydropyridine 3 and 2 acetyl 1 pyrroline 6 are the major contributors to the flavor of bread. Both compounds, having a pronounced cracker-like aroma, possess a great potential for use in bread, rice and bakery products in general. This pronounced potential especially originates from the extremely low odor threshold values, i.e. the minimum physical intensity detection where the subject is not required to identify the stimulus but just to detect the existence of the stimulus [R. Teranishi, R.G. Buttery, N. Schamp, Flavour Science and Technology, Ed. M. Martens, G.A. Dalen, H. Russwurm, Jr., p. 515, J. Wiley and Sons, Ltd. (1987)]. The odor threshold values of 2-acetyl-1,4,5,6-tetrahydropyridine 3 and 2-acetyl-1-pyrroline 6 were established as 1.4 ppb and 0.1 ppb, respectively [R. Teranishi et al., Flavour Science and Technology, P. 515, J. Wiley (1987)]. Because of these interesting flavor characteristics, both compounds are of major interest for the flavor industry.

The purpose of the present invention is to develop an attractive straightforward process for producing flavor components for bread and rice from very cheap basic chemicals.

The new process is characterized in that it comprises the following steps :

a) Synthesis of 2-cyano-1-azacycloalkanes by oxidation of cyclic amines to cyclic imines and subsequent cyanation in situ of the cyclic imines according to the following scheme :

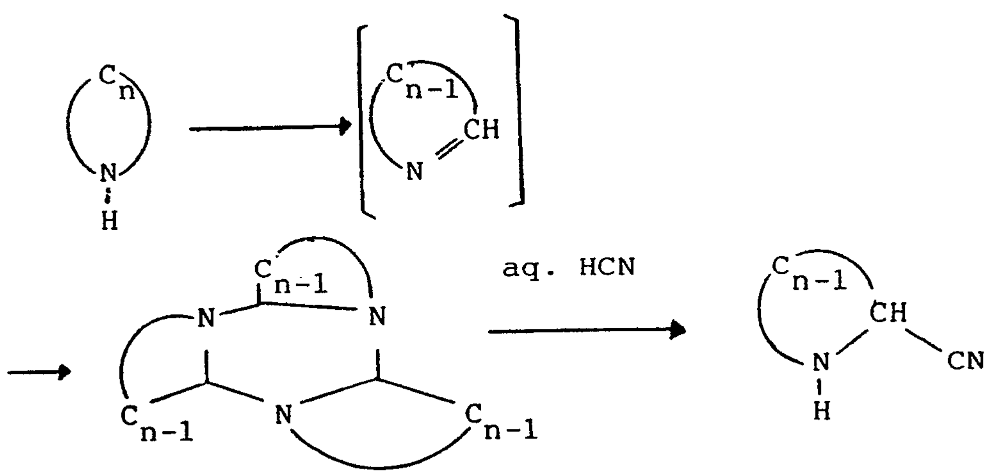


b) Synthesis of 2-cyano-1-aza-1-cycloalkenes by oxidation of 2-cyano-1-azacycloalkanes.

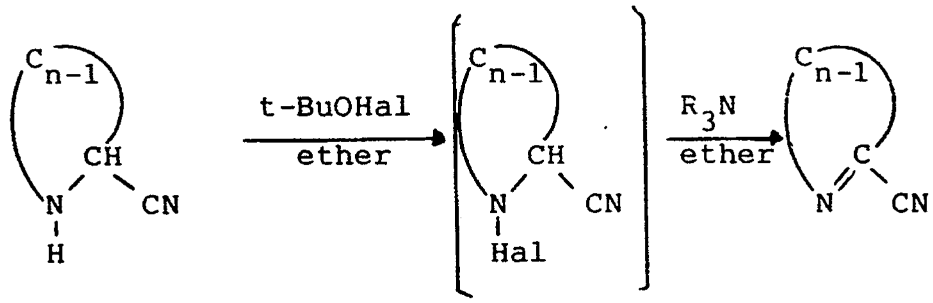


c) Synthesis of 2-acetyl-1-aza-1-cycloalkenes by a Grignard reaction.

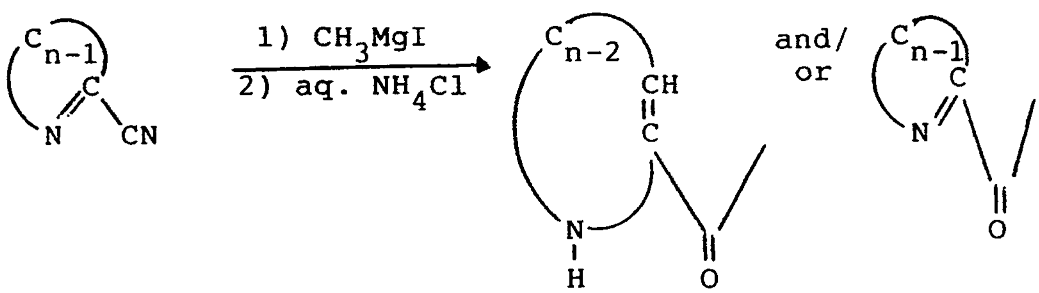


wherein

n = 4 or 5
Hal = Cl, Br
R = $CH_3$ or $C_2H_5$

Due to the ease of operation and the purity of the materials obtained, this synthesis can certainly be adapted for large scale preparations of these important cracker-like flavors.

This synthetic procedure utilizes cheap, basic chemicals and is easy to run. The end product is obtained free of side products.

In a first route, step 1 comprises direct cyanation in situ of the product obtained by oxidation of cyclic amines with t-butylhypohalite in ether at 0°C during 30 minutes and subsequent reaction of the N-halo cyclic amines with sodium methoxide in methanol according to the following scheme :

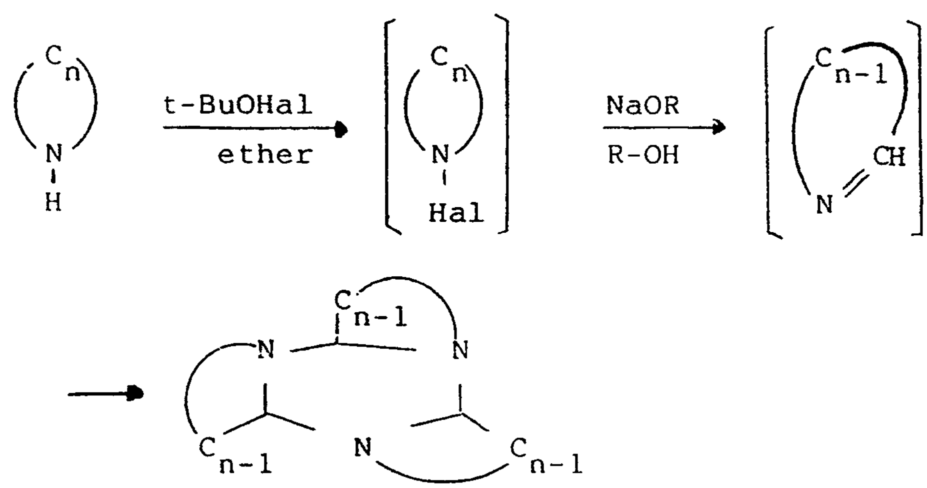


In an alternative route, step 1 comprises cyanation in situ of the product obtained by oxidation of cyclic amines by means of aqueous sodium peroxodisulfate under catalytic influence of silver nitrate.

A. Example 1 :

Synthesis of 2-Acetyl-1,4,5,6-tetrahydropyridine 3

A.1. Synthesis of 2-Cyanopiperidine 11

0.2 Mol of piperidine 7 in 130 ml dry ether was treated dropwise with 0.2 mol t-butylhypochlorite at 0° C. Stirring was continued at this temperature for 30 minutes. Three fourth of the solvent was evaporated in vacuo (t° <30° C) and the residual product (N-chloropiperidine 8 in ether) was triturated with 0.26 mol of 2N sodium methoxide in methanol. In most cases, a vigorous reaction started after several minutes. After this vigorous reaction ceased or after an additional 20 minutes at room temperature, the mixture was stirred under reflux for 45 minutes. The precipitate was then filtered and washed with little dry methanol, after which the solvent was removed in vacuo. The residual light-yellow solid (tripiperideine 10) [G.P. Claxton, L. Allen, J.M. Grisar, Org. Synth., Vol 56, 118 (1977)] was directly treated with aqueous hydrogen cyanide, prepared from 0.4 mol potassium cyanide and 0.4 mol 4N HCl (hood!). If necessary, some additional HCl 12N was added dropwise in order to acidify the solution ( water bath cooling). The mixture was stirred at ambient temperature for 1 h. Sodium hydroxide pellets were added under cooling till alkaline. The aqueous phase was extracted three times with dichloromethane, the organic phases were dried ($MgSO_4$) and evaporated in vacuo to leave a clear oil, which was distilled in vacuo to afford pure 2-cyanopiperidine 11 - (65% yield), bp. 91-95° C/12 mmHg [Lit. bp. 90-92° C/12 mmHg; H. Böhme, H. Ellenberg, O.-E. Herboth, W. Lehners, Chem. Ber., vol.92, 1608 (1959)].

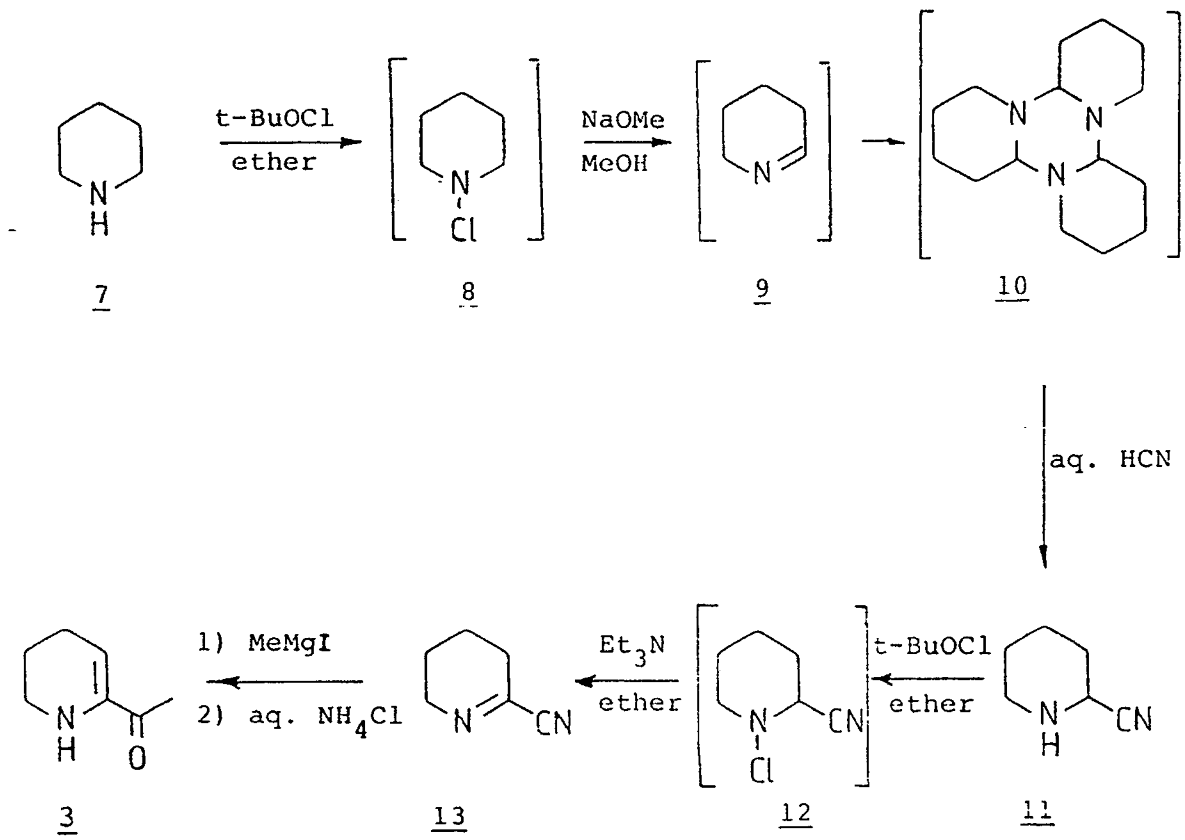


A.2. Synthesis of 2-Cyano-3,4,5,6-tetrahydropyridine 13

A solution of 4.4 g (0.04 mol) of 2-cyanopiperidine 11 in 50 ml dry ether was treated with 4.34 g (0.04 mol) of t-butylhypochlorite at 0° C. After stirring 1 h at this temperature, 4.04 g (0.04 mol) of triethylamine was added and stirring was continued during overnight. The precipitate was filtered, washed with dry ether, and evaporated without heating in vacuo. The remaining t-butanol was evacuated under high vacuum (0.05 mmHg) for 30 minutes. The remaining imidoyl cyanide 13 was obtained in quantitative yield and characterized by spectroscopic methods ($^1H$ NMR, $^{13}C$ NMR, IR, MS). It was essential to avoid the use of elevated temperatures as otherwise tautomerism to the corresponding enamine occurred. This tautomerism seemed to have a negative influence on the elaboration of this compound in the next step.

A.3. Synthesis of 2-Acetyl-1,4,5,6-tetrahydropyridine 3

Freshly prepared 2-cyano-3,4,S,6-tetrahydropyridine 13 (0.04 mol), obtained as described above in quantitative yield, was dissolved in 20 ml dry ether and this solution was added dropwise to a vigorously stirred solution of freshly prepared methylmagnesium iodide in ether at -20° C (the Grignard reagent was prepared from 0.12 gramatom magnesium turnings and 0.12 mol methyl iodide in 120 ml ether under reflux for 1 hour). After complete addition of the imidoyl cyanide 13, stirring was continued for 1 hour. The supernatans was cautiously poured into a stirred and ice-cold aqueous ammonium chloride solution in an erlenmeyer. The viscous residue remaining in the reaction flask was triturated with the ice-cold mixture from the erlenmeyer. The clear organic and aqueous layers were stirred vigorously for 20 minutes at ambient temperature. The organic layer was isolated, and the aqueous phase twice extracted with ether. The combined ether extracts were dried ($MgSO_4$) for 30 minutes. The drying agent was filtered and replaced for a fresh portion magnesium sulfate. After drying overnight at 5° C, the drying agent was filtered and the solvent removed in vacuo without heating to afford 2.2 g light-yellow oil, which consisted of pure (purity ≥ 98%) 2-acetyl-1,4,5,6-tetrahydropyridine 3 (GLC, $^1H$ MMR, $^{13}C$ NMR, IR, MS) (44% yield). Freshly prepared compound 3 occurred as a 4:1 mixture of the imino form and the enamino form ($^1H$ NMR, $CDCl_3$). On standing, this ratio gradually changed to a ratio in favor of the enamino form (up to 1:2). The freshly prepared bread flavor compound 3 is rather labile in neat form and should therefore be kept in diluted solution (pentane, $CH_2Cl_2$, ...) at -20° C (stable for years). Alternatively the stable hydrochloride salt can be prepared by reacting 3 with dry hydrogen chloride in ether [G. Büchi et al., J. Org. Chem., vol.36, 609 (1971)]. This hydrochloride is stable for years as a free flowing powder kept at -20° C protected from moisture.

B. Example 2 :

Synthesis of 2-Acetyl-1-pyrroline 6

B.1. Synthesis of 2-Cyanopyrrolidine 16

Pyrrolidine 14 was oxidized by aqueous sodium peroxodisulfate to 1-pyrroline trimer 15 (70-78% yield) as described in the literature [K. Ogawa, Y. Nomura, Y. Takeuchi, S. Tomoda, J. Chem. Soc. Perkin I, 3031 (1982)]. The crude trimer 15 was cyanated to 2-cyanopyrrolidine 16 in essentially the same way as described for compound 11 (see A.1.). 2-Cyanopyrrolidine 16 was obtained as a colorless oil, bp. 70-75° C/14 mmHg (65% yield) [Lit. bp. 168-172° C; R. Bonnett, V.M. Clark, A. Giddey, A. Todd, J. Chem. Soc., 2087 (1959)].

B.2. Synthesis of 2-Cyano-1-pyrroline 17

Imidoyl cyanide 17 was prepared in exactly the same way as described for imidoyl cyanide 13 (see A.2). Compound 17 was obtained in 90-95% yield and used immediately in the next step, after complete characterization by means of $^1H$ NMR, $^{13}C$ NMR, IR and MS.

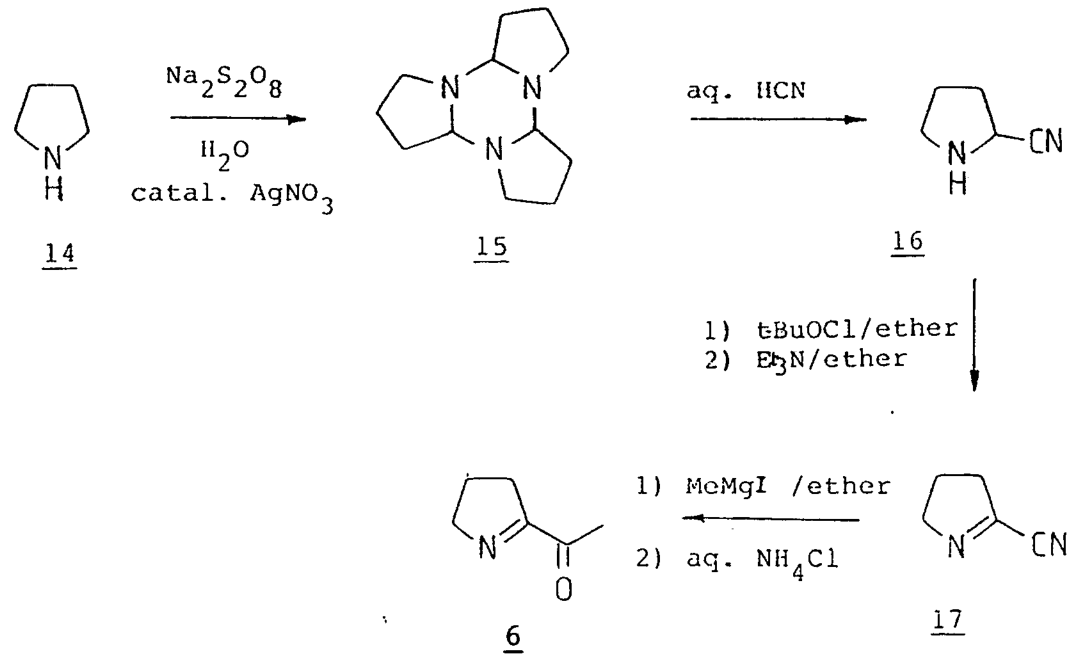


B.3. Synthesis of 2-Acetyl-1-pyrroline 6

The rice flavor compound 6 was prepared in exactly the same way as described in detail for the synthesis of the bread flavor component 3 (see A.3.). Compound 6 was obtained as a clear light-yellow oil (purity ≥ 96%) which darkened rapidly on standing at room temperature in neat form (yield 40%). Compound 6 was characterized by the usual spectrometric methods ($^1H$ NMR, $^{13}C$ NMR, IR, MS). It should be stressed that, contrary to compound 3, the rice flavor component 6 exclusively occurs as the imine form. The compound is preferably kept in dilute solution (pentane, dichloromethane) at -20° C. After an inital decantation from a small amount of dark viscous liquid (one week at -20° C), the clear solution is stable for several months at -20° C (up to now, we observed a good stability over a period of two years).

## Claims

1. Process for preparing 2-acetyl-1-aza-1-cycloalkenes usefull as flavor components for bread and rice, characterized in that it comprises following steps :

   a) Synthesis of 2-cyano-1-azacycloalkanes by oxidation of cyclic amines to cyclic imines and subsequent cyanation in situ of the cyclic imines according to the following scheme :

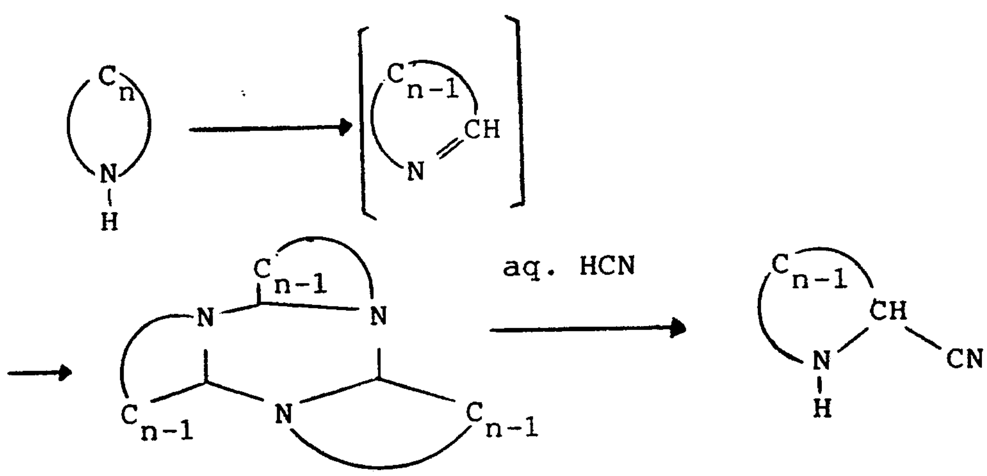


   b) Synthesis of 2-cyano-1-aza-1-cycloalkenes by oxidation of 2-cyano-1-azacycloalkanes.

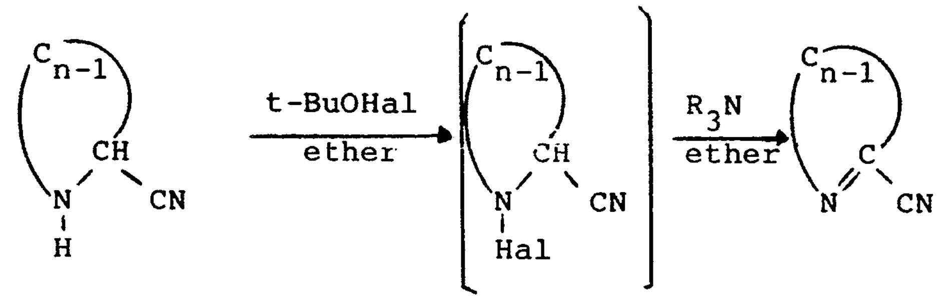


c) Synthesis of 2-acetyl-1-aza-1-cycloalkenes by a Grignard reaction.

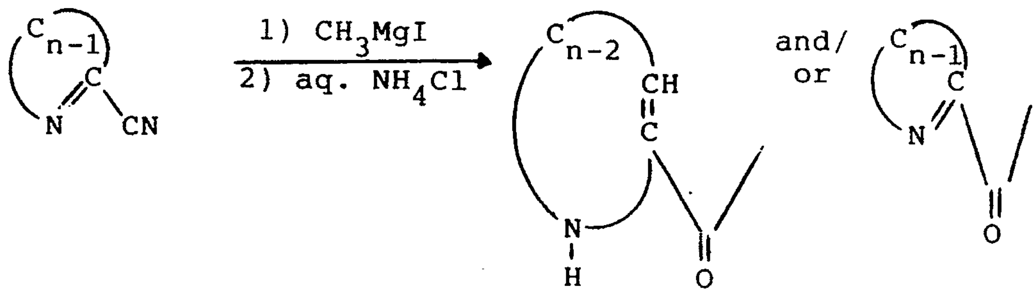


wherein

| | |
|---|---|
| n | = 4 or 5 |
| Hal | = Cl, Br |
| R | = $CH_3$ or $C_2H_5$ |

**2.** Process according claim 1, characterized in that it comprises direct cyanation in situ of the product obtained by oxidation of cyclic amines with t-butylhypohalite in ether at 0° C during 30 minutes and subsequent reaction of the N-halo cyclic amines which are triturated with sodium methoxide in methanol according to the following scheme :

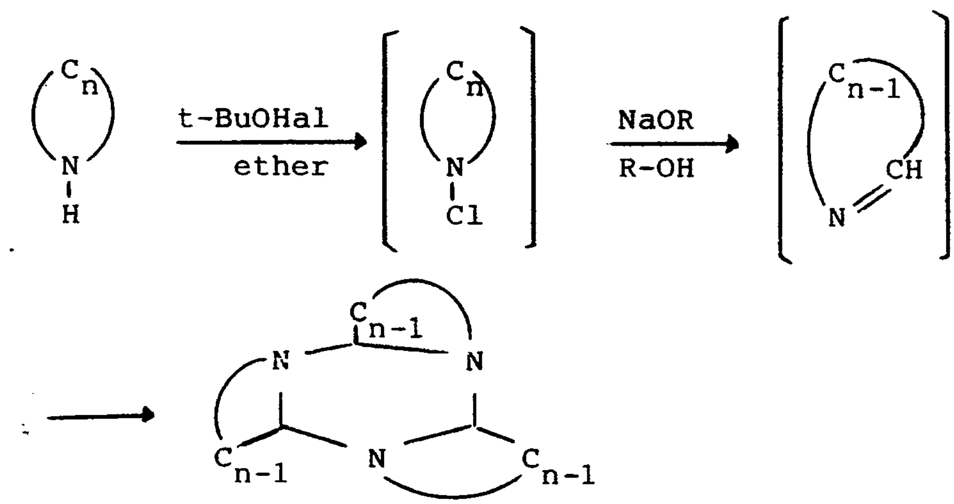


**3.** Process according claim 1 or 2, characterized in that it comprises cyanation in situ of the product obtained by oxidation of cyclic amines by means of aqueous sodium peroxodisulfate under addition of silver nitrate as catalyst.

European Patent Office

# EUROPEAN SEARCH REPORT

Application Number

EP 90 87 0004

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | US-A-4 522 838 (BUTTERY et al.)<br>* Example I; claims *<br>--- | 1-3 | C 07 D 207/20<br>C 07 D 211/70 //<br>A 23 L 1/226 |
| D,A | US-A-3 725 425 (HUNTER et al.)<br>* Example 1; claims *<br>--- | 1-3 | |
| D,A | US-A-3 620 771 (HUNTER et al.)<br>* Example 1; claims *<br>--- | 1-3 | |
| D,A | THE JOURNAL OF ORGANIC CHEMISTRY, vol. 36, no. 1, 15th January 1971, pages 609-610; G. BUCHI et al.: "Synthesis of 2-acetyl-1,4,5,6-tetrahydropyridine, a constituent of bread aroma"<br>* Entire publication *<br>----- | 1-3 | |
| | | | TECHNICAL FIELDS SEARCHED (Int. Cl.5) |
| | | | C 07 D 207/00<br>C 07 D 211/00 |

The present search report has been drawn up for all claims

| Place of search | Date of completion of the search | Examiner |
|---|---|---|
| THE HAGUE | 06-09-1990 | KISSLER B.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

# ANNEX TO THE EUROPEAN SEARCH REPORT ON EUROPEAN PATENT APPLICATION NO.

EP 90 87 0004

This annex lists the patent family members relating to the patent documents cited in the above-mentioned European search report.
The members are as contained in the European Patent Office EDP file on 17/09/90
The European Patent Office is in no way liable for these particulars which are merely given for the purpose of information.

| Patent document cited in search report | Publication date | Patent family member(s) | Publication date |
|---|---|---|---|
| US-A- 4522838 | 11-06-85 | None | |
| US-A- 3725425 | 03-04-73 | None | |
| US-A- 3620771 | 16-11-71 | None | |



For more details about this annex : see Official Journal of the European Patent Office, No. 12/82